(19) Europäisches Patentamt
European Patent Office
Office européen des brevets

(11) Veröffentlichungsnummer: **0 234 013 B1**

(12) # EUROPÄISCHE PATENTSCHRIFT

(45) Veröffentlichungstag der Patentschrift: **19.09.90**

(51) Int. Cl.⁵: **C07C 51/16**, C07C 53/21, C07C 55/32

(21) Anmeldenummer: **86116766.6**

(22) Anmeldetag: **02.12.86**

(54) **Verfahren zur Herstellung von Perfluorcarbonsäuren.**

(30) Priorität: **26.02.86 DE 3606174**

(43) Veröffentlichungstag der Anmeldung:
**02.09.87 Patentblatt 87/36**

(45) Bekanntmachung des Hinweises auf die Patenterteilung:
**19.09.90 Patentblatt 90/38**

(84) Benannte Vertragsstaaten:
**DE FR GB IT**

(56) Entgegenhaltungen:
**DE-A- 1 768 050**

**THE JOURNAL OF THE AMERICAN OIL CHEMISTS' SOCIETY, Band 54, Nr. 11, November 1977, Seiten 858A-861A, Champaign, Illinois, US; T.A. FOGLIA et al.: "Oxidation of alkenes with use of phase transfer catalysis"**
**MONATSHEFTE FÜR CHEMIE, Band 110, Nr. 6, November/Dezember 1979, Seiten 1471-1473, Springer-Verlag, Wien, AT; D. SCHOLZ: "Neue Synthesemethoden 2. Milde Einphasenoxidation von Aldehyden zu Carbonsäuren"**

(73) Patentinhaber: **HOECHST AKTIENGESELLSCHAFT, Postfach 80 03 20, D-6230 Frankfurt am Main 80(DE)**

(72) Erfinder: **Von Werner, Konrad, Dr., Buch 1 1/2, D-8261 Halsbach(DE)**
Erfinder: **Probst, Anton, Gerhart-Hauptmann-Strasse 5, D-8269 Burgkirchen(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 234 013 B1

ACTORUM AG

## Beschreibung

Die Erfindung betrifft ein Verfahren zur Herstellung von perfluorierten aliphatischen Carbonsäuren gemäß Patentanspruch 1.

Es sind verschiedene Methoden zur Gewinnung von Perfluorcarbonsäuren bekannt. Die Elektrofluorierung von Carbonsäurechloriden oder -fluoriden ist beispielsweise von D. Lines und H. Sutcliffe; J. Fluorine Chem. 17 (1981) S 423, und in der dort zitierten Literatur beschrieben. Sie liefert jedoch bei längerkettigen Säuren geringe Ausbeuten wegen der konkurrierenden Bildung von perfluorierten Ethern und Alkanen. Die aus US-PS 4,400,325 bekannte Oxidation von Iod-Perfluoralkanen mit Oleum gibt zwar hohe Ausbeuten, erfordert aber eine spezielle Aufarbeitung zur Rückgewinnung von Iod. Aus US-PS 4,221,734 ist die Herstellung von Perfluorcarbonsäuren aus Iod-Perfluoralkanen, Zink und Kohlendioxid bekannt, diese gelingt nur in aprotischen Lösungsmitteln nach einer aufwendigen Aktivierung des Zinks. In US-PS 4,138,417 ist die Oxidation von Alkenen der Formel $R_FCH{=}CH_2$ mit Ozon beschrieben, diese ist umständlich, da sie eine Behandlung des primär gebildeten Gemischs aus perfluorierten Aldehyden und Ozoniden mit einem weiteren Oxidationsmittel erfordert. Nach C. Guizard und C. G. Beguin; J. Fluorine Chem. 13 (1979) S 175, gelingt die Oxidation der Olefine der Formel $R_FCH{=}CH_2$ auch mit verschiedenen Oxidationsmitteln unter Verwendung von Ruthenium-dioxid als Katalysator, jedoch wird hierbei eine aufwendige Rückgewinnung des teuren Katalysators notwendig.

In JP-AS (Kokoku) 68-29,129 ist die Herstellung von β-Bromtetrafluorpropionsäure aus 3,3,4,4-Tetrafluor-4-brom-1-buten unter Verwendung von Kaliumpermanganat und Wasser beschrieben. Wendet man dieses Verfahren auf Alkene mit längerer perforierter Alkylkette an, so verläuft die Reaktion unkontrollierbar, das heißt, es tritt zunächst keine Reaktion ein, später springt die Reaktion an, aber so heftig, daß sie nicht mehr beherrscht werden kann, wie nachfolgender Vergleichsversuch zeigt. Diese Schwierigkeit kann zwar möglicherweise in einigen Fällen durch Verwendung von stark verdünnten, wäßrigen Permanganat-Lösungen vermindert werden, doch sind dann lange Reaktionszeiten erforderlich und nur eine äußerst ungünstige Raum-Zeit-Ausbeute erzielbar.

In DE-OS 1 768 050 ist unter anderem die Verwendung von quaternären Ammoniumsalzen bei der Oxidation von nicht fluorhaltigem Alken und Diarylalken beschrieben. Wie die Beispiele 13 und 14 zeigen, bewirkt schon ein geringer Zusatz eines quaternären Ammoniumsalzes eine stürmische Reaktion mit beträchtlicher Wärmeentwicklung.

Nach T. A. Foglia, P. A. Barr und A. J. Malloy in "The Journal of the American Oil Chemists' Society", Vol. 54 (1977) Seite 858A, rechte Spalte sowie Seite 859A, Tabelle I kann aus 9-Octadecen mit einer Lösung von Kaliumpermanganat in Wasser unter Zusatz einer Lösung eines quaternären Ammoniumsalzes in Dichlormethan oder Benzol Pelargonsäure (charakterisiert durch ihren Methylester) mit 50 bis 80 % Ausbeute erzeugt werden, wobei ein Molverhältnis 9-Octadecen/quaternäres Ammoniumsalz von 1 : 0,3 angewendet wird.

Das weiter unten beschriebene erfindungsgemäße Verfahren ermöglicht es, Perfluoralkylalkene mit höheren Ausbeuten ohne Verwendung organischer Lösungsmittel mit deutlich geringeren Zusätzen von quaternärem Ammoniumsalz zu erzeugen, wobei im Gegensatz zur Lehre der DE-OS 1 768 050 eine stürmische Reaktion, die ohne Zusatz von quaternärem Ammoniumsalz beobachtet wird, gedämpft wird, so daß ein gleichmäßiger Reaktionsverlauf erfolgt. Die erfindungsgemäß, ohne organische Lösungsmittel erzielte hohe Ausbeute an Perfluorcarbonsäuren war nicht zu erwarten, da die quaternären Ammoniumsalze in den als Ausgangsstoffe verwendeten Perfluoralkylalkenen, die ausschließlich die organische Phase bilden, nur sehr wenig löslich sind.

Wie bereits oben ausgeführt, wurde ein Verfahren gefunden, das es ermöglicht, insbesondere längerkettige perfluorierte aliphatische Carbonsäuren aus den entsprechenden Perfluoralkylalkenen in glatt verlaufender, leicht zu beherrschender Reaktion mit vergleichsweise guten Raum-Zeit-Ausbeuten zu erzeugen. Es handelt sich um ein Verfahren zur Herstellung von Perfluorcarbonsäuren durch Oxidation von Perfluoralkylalkenen mit Permanganat in wäßriger Lösung, gegebenenfalls bei erhöhter Temperatur, das dadurch gekennzeichnet ist, daß man 1/p mol einer Verbindung der Formel

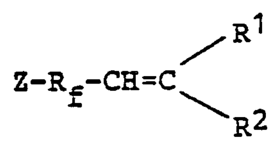

(I),

worin bedeuten:

$R_f$ eine geradkettige, verzweigte oder cyclische Perfluoralkylengruppe mit 6 bis 18 C-Atomen;

Z = F; Cl; Br oder

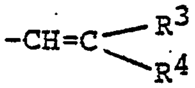

$R^1$, $R^2$, $R^3$, und $R^4$ jedes für sich ein Wasserstoffatom oder eine Alkylgruppe mit 1 bis 5 C-Atomen, wobei ferner p die Anzahl der Doppelbindungen im Molekül der Formel I ist, in Gegenwart von 0,001 bis 0,1 mol mindestens eines Alkyl- und/oder Arylalkyl-Gruppen enthaltenden quaternären Ammoniumsalzes umsetzt, nach Beendigung der Reaktion die Mischung ansäuert, das entstandene Mangan-IV-oxid abtrennt oder durch Reduktion zum Mangan-II-salz in Lösung bringt, die entstandene Perfluormono- oder -di-carbonsäure abtrennt und, sofern erforderlich, weiter reinigt.

In der Verbindung der Formel I kann $R_f$ eine geradkettige oder verzweigte Perfluoralkylen-Gruppe sein. Verzweigt im Sinne der Erfindung sind auch cyclische Perfluoralkylen-Gruppen. Sofern diese mehr als 18 C-Atome aufweisen, sind die Ausgangsprodukte schwerer zugänglich, die Reaktionsdauer nimmt zu und die Aufarbeitung des Reaktionsgemisches wird häufig schwieriger. Wenn $R_f$ eine Perfluoralkylen-Gruppe mit weniger als 6 C-Atomen ist, kann die erfindungsgemäße Reaktion ebenfalls angewendet werden, doch mit weniger Vorteil im Vergleich zu Methoden nach dem bekannten Stand der Technik. Wegen der guten Zugänglichkeit der Ausgangsprodukte und Verwendbarkeit der erzeugten Perfluorcarbonsäuren werden Verbindungen der Formel I bevorzugt, in denen $R_f$ 6 bis 10 C-Atome aufweist. Das gleiche gilt auch für Verbindungen der Formel I, in denen Z = F; Cl oder Br ist. Ferner werden solche Verbindungen der Formel I bevorzugt eingesetzt, in denen $R^1$, $R^2$, und sofern vorhanden, $R^3$ und $R^4$, jedes für sich, ein Wasserstoffatom oder eine Methyl- oder eine Ethylgruppe ist, wobei insbesondere gute Ergebnisse erzielt werden, wenn die genannten Radikale Wasserstoffatome sind.

Wenn p die Anzahl der Doppelbindungen im Molekül der Formel I ist, wird die Reaktion in Gegenwart von 0,001 bis 0,1 mol mindestens eines Alkyl- und/oder Arylalkyl-Gruppen enthaltenden quaternären Ammoniumsalzes je 1/p mol der Verbindung der Formel I durchgeführt. Bei unter 0,001 mol quaternärem Ammoniumsalz wird die Reaktion nicht mehr beherrschbar oder muß in sehr großer Verdünnung vorgenommen werden, wodurch die Raum-Zeit-Ausbeute sehr ungünstig wird. Mehr als 0,1 mol quaternäres Ammoniumsalz je 1/p mol der Verbindung der Formel I kann zwar verwendet werden, jedoch wird dadurch kein zusätzlicher Effekt mehr beobachtet, der den erhöhten Aufwand lohnen würde. Vorzugsweise wird im Bereich von 0,01 bis 0,05 mol quaternäres Ammoniumsalz gearbeitet.

Als quaternäre Ammoniumsalze eignen sich beispielsweise solche mit 4 gleichen Alkyl- oder Arylalkyl-Gruppen,wie beispielsweise Tetrabutyl-,Tetrahexyl- oder Tetrabenzyl-Ammoniumsalze oder solche, die einen längerkettigen und drei gleiche kurzkettige Alkylreste aufweisen, wie beispielsweise Palmityltrimethyl- oder Lauryltriethylsalze. Besonders gute Ergebnisse werden erhalten, wenn als quaternäres Ammoniumsalz mindestens eine der Verbindungen der Formeln $[(R^5)_2(R^6)_2N]^+X^-$ und $[(R^5)_3R^6N]^+X^-$ eingesetzt werden, wobei in diesen Formeln bedeuten: $R^5$ einen Alkyl- oder Arylalkylrest mit 8 bis 20 C-Atomen; $R^6$ einen Alkylrest mit 1 bis 3 C-Atomen und $X^-$ = den 1/mten Teil eines m-wertigen Ions, ausgewählt aus der Gruppe $Cl^-$; $Br^-$; $R^8SO_4^-$; $SO_4^{2-}$; $PO_4^{3-}$; $R^8PO_4^{2-}$; $(R^8)_2PO_4^{1-}$ $R^7SO_3^{1-}$; $R^7PO_3^{2-}$; $(R^7)_2PO_3^{1-}$, worin bedeuten $R^7$ = Alkyl- mit 1 bis 4 C-Atomen und $R^8$ = H oder Alkyl mit 1 bis 4 C-Atomen. Anstelle der genannten salzbildenden Anionen können auch noch andere Anionen, vorzugsweise von anorganischen Säuren verwendet werden, die von Permanganat-Ionen nicht oxidiert werden. Es können auch Mischungen von mehreren quaternären Ammoniumsalzen Verwendung finden.

Die Verbindung der Formel I, auch Mischungen von mehreren Verbindungen dieser Formel sind geeignet, werden mit Permanganat in wäßriger Lösung oxidiert. Hierzu werden zweckmäßig Permanganate eingesetzt, die in Wasser löslich sind, das heißt, von denen sich mindestens 0,5 g in 1 $dm^3$ Wasser bei 20 °C lösen. Hierfür kommen in erster Linie die Permanganate von Metallen der 1. und 2. Gruppe des Periodensystems der Elemente in Frage, wegen ihrer leichten und kostengünstigen Beschaffbarkeit, insbesondere Natrium- oder Kaliumpermanganat.

Die Reaktion wird bei 10 bis 100 °C durchgeführt. Eine Kühlung unter 10 °C ist im allgemeinen nicht erforderlich, auch verläuft bei niedrigeren Temperaturen die Reaktion unnötig langsam. Bei über 100 °C treten vermehrt unerwünschte Nebenreaktionen ein, ferner müßte durch die Verwendung von druckstabilen Apparaturen ein Aufwand getrieben werden, der sonst nur in Sonderfällen, wenn niedrigsiedende Lösungsmittel mitverwendet werden, erforderlich ist. Vorzugsweise wird bei 20 bis 60 °C gearbeitet.

Es ist nicht erforderlich, soviel Wasser zu verwenden, daß sich das gesamte Permanganat löst. Es reicht im allgemeinen vollständig aus, je Gramm eingesetzte Verbindung der Formel I 1 bis 10 Gramm, vorzugsweise 2 bis 5 Gramm Wasser zu verwenden. Ein bei Beginn der Reaktion etwa vorhandener ungelöster Bodenkörper aus Permanganat löst sich während der Reaktion nach. Durch Inbewegunghalten der Reaktionsmischung, beispielsweise durch Rühren, werden der Nachlösevorgang und die Einstellung einer gleichmäßigen Temperatur des Reaktionsgemisches durch Kühlung begünstigt. Eine größere Wassermenge als 10 g je 1 g der Verbindung der Formel I kann zwar verwendet werden, doch wird hierdurch die Raum-Zeit-Ausbeute verschlechtert, ohne daß günstige Effekte beobachtet werden, die diesen Nachteil ausgleichen würden.

Die Menge des zu verwendenden Permanganats hängt ab von der Art der Verbindung der Formel I. Wenn in letzterer Verbindung bedeuten Z = F, Cl oder Br sowie $R^1$ und $R^2$ = H, werden vorzugsweise auf

3 mol dieser Verbindung mindestens, 10 mol Permanganat-Ionen verwendet. Bedeuten dagegen in der Verbindung der Formel I $Z = -CH=CH_2$ sowie $R^1$ und $R^2$ = H, werden vorzugsweise auf 3 mol dieser Verbindung mindestens 20 mol Permanganat-Ionen eingesetzt.

Wenn in der Verbindung der Formel I bedeuten Z = F, Cl oder Br sowie $R^1$ und $R^2$ = Alkyl mit 1 bis 5 C-Atomen, werden vorzugsweise auf 1 mol dieser Verbindung mindestens 2 mol Permanganat-Ionen angewendet. Bedeuten dagegen in der Verbindung der Formel I

$$Z = -CH=C\begin{matrix} R^3 \\ R^4 \end{matrix}$$

sowie $R^1$, $R^2$, $R^3$ und $R^4$,unter sich gleich oder verschieden, = Alkyl mit 1 bis 5 C-Atomen, werden vorzugsweise auf 1 mol dieser Verbindung 4 mol Permanganat-Ionen eingesetzt.

Die Menge der Permanganat-Ionen kann in allen genannten Fällen größer sein, sollte jedoch im allgemeinen das 1,5fache der genannten Mindestmenge nicht überschreiten, andernfalls wird die Reaktion unnötig teurer und auch Nebenreaktionen begünstigt. Besonders gute Ergebnisse werden erhalten, wenn die Menge der Permanganat-Ionen das 1,3fache der weiter oben genannten Mindestmenge nicht übersteigt.

Für die erfindungsgemäße Reaktion ist außer Wasser im allgemeinen kein weiteres Lösungsmittel erforderlich. In Sonderfällen, beispielsweise wenn die Verbindung der Formel I unter den gewählten Reaktionsbedingungen nicht flüssig ist, können vorteilhaft durch Permanganat-Ionen nicht oxidierbare Lösemittel mitverwendet werden, um die Verbindung der Formel I ganz oder teilweise in den flüssigen Zustand überzuführen. Als solche Lösungsmittel sind beispielsweise geeignet niedere Kohlenwasserstoffe, deren Wasserstoffatome ganz oder zum größten Teil durch Fluor oder Chloratome ersetzt sind, wobei in einem Molekül auch Fluor- und Chloratome gemeinsam vorkommen können, mit der Maßgabe, daß diese niederen halogenierten Kohlenwasserstoffe einen Siedepunkt bei normalem Atmosphärendruck von mindestens 30 °C aufweisen. Beispielhaft seien genannt Dichlormethan, Chloroform oder 1,1,2-Trifluor-1,2,2-trichlorethan.

In den meisten Fällen wird die neue Reaktion unter normalem Atmosphärendruck durchgeführt. Wie oben bereits erwähnt, kann es bei Verwendung enstprechender Lösemittel und Reaktionstemperaturen im oberen Bereich notwendig sein, unter dem autogenen Druck des Reaktionsgemisches der bis zu etwa 1 MPa betragen kann, zu arbeiten.

Die Dauer der Reaktion hängt von den Reaktionspartnern und der gewählten Temperatur ab. Im allgemeinen ist sie nach 0,5 bis 15 h, vorzugsweise nach 1 bis 8 h beendet.

Sofern für die Reaktion sehr reine Ausgangssubstanzen, insbesondere eine sorgfältig gereinigte Verbindung der Formel I,verwendet werden, fällt das gebildete Mangan-IV-oxid in gut filtrierbarer Form an und kann durch Heißfiltration aus dem Reaktionsgemisch nach Beendigung der Reaktion abgetrennt werden. Bei Verwendung einer Verbindung der Formel I von nur technischer Reinheit macht die Filtration des Mangan-IV-oxids oft Schwierigkeiten, so daß es vorzugsweise in saurer Lösung bei einem pH von 0 bis 6,5 zu einer wasserlöslichen Mangan-II-Verbindung reduziert wird. Zum Einstellen und Halten der sauren Reaktion sind beispielsweise Schwefelsäure oder Phosphorsäure geeignet. Als Reduktionsmittel dienen beispielsweise Sulfite, Phosphite, phosphorige Säure, Oxalsäure oder Ameisensäure, bevorzugt wird Schwefeldioxid verwendet, da es kostengünstig ist, glatt reagiert und keine Probleme wegen zu starker Schaumbildung mit sich bringt. Das Schwefeldioxid kann gasförmig angewendet oder in der Reaktionsmischung gebildet werden.

Nach der Heißfiltration des Mangan-IV-oxids bzw. seiner Überführung in eine wasserlösliche Mangan-II-Verbindung wird die erzeugte Perfluoralkancarbonsäure aus dem Reaktionsgemisch abgetrennt. Bei schwer wasserlöslichen Carbonsäuren kann dies durch einfaches Abfiltrieren bzw. durch einfache Abtrennung der organischen Phase geschehen, wobei der Filterrückstand bzw. die flüssige organische Phase mit angesäuertem Wasser gewaschen werden. Zur Verbesserung der Ausbeute können die vereinigten wäßrigen Phasen mit einem geeigneten, mit Wasser nicht mischbaren Lösungsmittel bzw. Lösungsmittelgemisch extrahiert werden. Nach Abdampfen des oder der Lösungsmittel werden noch weitere Mengen Perfluoralkancarbonsäure erhalten. Geeignete Lösungsmittel sind beispielsweise Fluor und/oder Chlor bzw. Brom enthaltende Kohlenwasserstoffe mit 1 bis 3 C-Atomen, die nicht mehr als 1 H-Atom je C-Atom enthalten, Di-alkylether mit insgesamt 4 bis 8 C-Atomen oder mit Wasser nicht mischbare Ketone. Sofern die erzeugte Perfluoralkancarbonsäure gut wasserlöslich ist, wird zweckmäßig die Reaktionsmischung nach Abtrennung oder Reduktion des Mangan-IV-oxids zur Trockne verdampft, der Rückstand falls erforderlich beispielsweise mit Schwefelsäure angesäuert und die Perfluoralkansäure durch Destillation, oder, wie oben beschrieben, durch Extraktion abgetrennt und gewonnen.

Für viele Anwendungszwecke ist die Reinheit der so erhaltenen Perfluoralkylcarbonsäure ausreichend. Falls erforderlich, kann sie durch übliche Methoden wie beispielsweise fraktionierte Destillation, gegebenenfalls unter vermindertem Druck, oder Umkristallisieren, beispielsweise aus Toluol oder Chloroform, weiter gereinigt werden.

Die erfindungsgemäß erzeugten Perfluoralkylcarbonsäuren und insbesondere ihre Salze wie Alkali-oder Ammoniumsalze, können vielseitig verwendet werden, beispielsweise als Tenside zur Polymerisation von insbesondere fluorhaltigen Monomeren, Flußmittel zum Löten und als Gleitmittel. Sie dienen auch als Vorprodukte zur Herstellung von fluorierten Verbindungen für Feuerlöschmittel.

Wie bereits eingangs erwähnt, ermöglicht es das erfindungsgemäße Verfahren, Perfluoralkylcarbonsäuren auch größerer Kettenlängen in glatter und leicht beherrschbarer Reaktion mit hoher Reinheit und guten Raum-Zeit-Ausbeuten herzustellen.

Nachfolgende Beispiele sollen die Erfindung näher erläutern: Die erzeugten Perfluoralkylcarbonsäuren werden identifiziert durch $^{19}F$-Kernresonanz und durch Vergleich mit entsprechenden Verbindungen, die nach dem Stand der Technik aus Perfluoralkyliodid mittels Umsetzung mit Magnesium, anschließender Reaktion mit Kohlendioxid und Hydrolyse hergestellt wurden. Teilweise werden die Perfluoralkylcarbonsäuren in ihre Methylester umgewandelt und der gaschromatographischen Analyse unterworfen.

Beispiel 1

In einer Laborapparatur aus Glas werden zu einer Lösung von 4,0 g (0,01 mol) Trioctyl-methyl-ammonium-chlorid in 200 $cm^3$ entsalztem Wasser unter Rühren 125 g (0,8 mol) Kaliumpermanganat gegeben. Unter kräftigem Rühren werden langsam 70,6 g (0,2 mol) Perfluorhexyl-ethen zugetropft. Auf 3 mol der Verbindung der Formel I werden also 12 mol Permanganat-Ionen angewendet. 15 min nach Beginn der Zugabe ist die Temperatur auf 40 °C gestiegen; durch äußerliche Kühlung wird die Temperatur des Reaktionsgemisches auf 40 bis 45 °C gehalten, bis nach 2,5 h das gesamte Perfluorhexyl-ethen zudosiert ist. Danach wird noch 30 min gerührt, dann die Mischung auf 70 °C erhitzt und über ein geheiztes Druckfilter filtriert. Der Filterkuchen wird mit 100 $cm^3$ heißem Wasser gewaschen und die vereinigten Filtrate werden am Rotationsverdampfer zur Trockne eingedampft. Der so erhaltene Feststoff wird in einer Destillationsapparatur vorsichtig mit 200 $cm^3$ konzentrierter Schwefelsäure versetzt, wobei anfangs Schäumen durch Kohlendioxid-Entwicklung auftritt. Durch Destillation bei 1300 Pa (Kp = 75 bis 76 °C) werden 70,5 g Perfluorheptansäure erhalten. Die Ausbeute beträgt 96,8 %, die Reinheit (gaschromatographisch bestimmt) 99,8 %. Das Produkt erstarrt beim Abkühlen auf Raumtemperatur.

Beispiel 2

In einer Laborapparatur aus Glas werden zu einer Lösung von 1,83 g (0,004 mol) Didecyl-dimethyl-ammonium-methosulfat in 200 $cm^3$ entsalztem Wasser unter Rühren 68 g (0,43 mol) Kaliumpermanganat gegeben. Unter kräftigem Rühren werden langsam 74,8 g (0,2 mol) $C_6F_{13}$-$CH=C(CH_3)_2$ zugetropft. Es werden je mol der Verbindung der Formel I 2,15 mol Permanganat-Ionen verwendet. Es wird weiterverfahren wie unter Beispiel 1 beschrieben, wobei 69,75 g Perfluorheptansäure erhalten werden (Ausbeute 95,8%).

Beispiel 3

In einer Laborapparatur aus Glas werden zu einer Lösung von 1,82 g (0,004 mol) Didecyl-dimethyl-ammonium-hydrogensulfat in 200 $cm^3$ entsalztem Wasser unter Rühren 109 g (0,69 mol) Kaliumpermanganat gegeben. Unter kräftigem Rühren werden langsam 79,22 g (0,2 mol) $(CF_3)_2CF$-$(CF_2)_4$-$CH=CH_2$ innerhalb von 3 h bei einer Temperatur der Reaktionsmischung von 40 °C zugetropft. Auf 3 mol der Verbindung der Formel I werden 10,35 mol Permanganat-Ionen verwendet. Nach den 3 h wird unter fortgesetztem Rühren durch vorsichtige Zugabe von 65 $cm^3$ konzentrierter Schwefelsäure angesäuert. Nun werden 500 $cm^3$ einer kalten gesättigten Natriumsulfit-Lösung und anschließend noch 150 g Natriumsulfit-Hydrat zugegeben und bei 90 °C solange gerührt, bis das gesamte Mangan-IV-oxid in Lösung gegangen ist. Die untere, organische Phase wird abgetrennt und die auf Raumtemperatur abgekühlte wäßrige Phase mit Diethylether ausgeschüttelt. Verdampfen des Etherextraktes ergibt 50,8 g Rückstand, welcher mit der organischen Phase vereinigt und nach Zusatz von 50 $cm^3$ konzentrierter Schwefelsäure destilliert wird. Es werden 77,35 g $(CF_3)_2CF$-$(CF_2)_4$-$COOH$ mit einem Kp 83 bis 84 °C bei 1300 Pa als farblose Flüssigkeit erhalten. Dies entspricht einer Ausbeute von 93,4 %. Das Produkt hat eine Reinheit von 99,2 %.

Beispiel 4

Es wird verfahren wie in Beispiel 3 mit folgenden Unterschieden: anstelle von 200 $cm^3$ werden 300 $cm^3$ Wasser und anstelle der Verbindung der Formel $(CF_3)_2CF$-$(CF_2)_4$-$CH=CH_2$ werden 110,6 g (0,2 mol) Perfluordecylethen verwendet. Das sind auf 3 mol der Verbindung der Formel I 10,65 mol Permanganat-Ionen. Anstelle 40 °C wird die Reaktion bei einer Temperatur der Reaktionsmischung von 50 bis 55 °C während 3 h durchgeführt. Nach Zugabe von Schwefelsäure und Sulfit-Reaktion des Mangan-IV-oxids wie in Beispiel 3 beschrieben, wird nach Abkühlen auf Raumtemperatur die weiße, feste Rohsäure aus der rosa gefärbten Lösung abfiltriert und mit 100 $cm^3$ kaltem Wasser gewaschen. Nach Zusatz von 50 $cm^3$ konzentrierter Schwefelsäure wird das Produkt bei einem Druck von 1500 Pa und einer Temperatur von 132 bis 135 °C sublimiert. Es werden 99,1 g Perfluorundecansäure als weißer Feststoff mit einem Schmelzpunkt von 103 bis 104 °C erhalten. Das entspricht einer Ausbeute von 87,8 %.

Beispiel 5

In einem gummierten Kessel von 10 $dm^3$ Inhalt, der mit einem gummierten Rührer ausgerüstet ist, werden 15,25 g (0,049 mol) Dioctyl-dimethyl-ammoniumchlorid in 4250 $cm^3$ salzfreiem Wasser gelöst und 1354 g (8,57 mol) Kaliumpermanganat zugegeben. Die Mischung wird 10 min gerührt, dann auf 40 °C erwärmt. Anschließend werden bei einer Rührerdrehzahl von 600 UpM 1225 g technisches Perfluoroctylethen (Verunreinigungen: 9,0 Gew.-% $C_{10}F_{21}H$ und 1,5 % anderer Fluorverbindungen), welches 2,46 mol Perfluoroctylethen enthält, innerhalb 5 h zudosiert, wobei die Temperatur des Reaktionsgemisches durch Kühlung auf 55 °C gehalten wird. Auf 3 mol der Verbindung der Formel I werden 10,45 mol Permanganat-Ionen verwendet. Es wird noch eine weitere Stunde gerührt, nach 30 min ist dabei keine Kühlung mehr erforderlich, um die Temperatur von 55 °C zu halten. Nun werden 1285 g 96 %ige Schwefelsäure unter Rühren so langsam zugegeben, daß der durch Kohlendioxid-Entwicklung entstehende Schaum im Kessel nicht zu weit nach oben steigt. Unter fortgesetztem Rühren werden dann bei geschlossenem Abgasventil 650 g Schwefeldioxid-Gas innerhalb von 3,5 h in den Gasraum des Kessels eingeleitet, wobei die Temperatur der Mischung durch Kühlung auf 45 °C gehalten wird. Nach weiteren 30 min Rühren ist das Mangan-IV-oxid vollständig reduziert. Nun wird das Abgasventil geöffnet und ein Stickstoffstrom von 30 $dm^3$ pro Stunde in den Kessel eingeleitet, während der Kesselinhalt bei einer Rührerdrehzahl von 300 UpM auf 80 bis 90 °C aufgeheizt wird. Nach Abstellung von Heizung und Rührer wird 15 min gewartet und dann die noch ca. 70 °C heiße, flüssige untere organische Phase abgetrennt. Diese wird mit 350 g konzentrierter Schwefelsäure vermischt und im Vakuum über eine Kolonne destilliert. Bei 2400 Pa Druck beträgt der Siedepunkt 110 bis 111 °C. Es werden 970 g feste Perfluornonansäure vom Schmelzpunkt 57 bis 59 °C erhalten. Die Ausbeute beträgt 85,0 %. Nach gaschromatographischer Analyse besteht das Produkt zu 99,3 % aus Perfluornonansäure und 0,7 % Perfluoroctansäure.

Beispiel 6

In einer Laborapparatur aus Glas werden zu einer Lösung von 0,61 g (0,002 mol) Dioctyl-dimethylammoniumchlorid in 100 $cm^3$ entsaltem Wasser unter Rühren 110,6 g (0,7 mol) Kaliumpermanganat gegeben. Unter kräftigem Rühren werden nun langsam 35,4 g (0,1 mol) 3,3,4,4,5,5,6,6,7,7,8,8-Dodecafluor-1,9-decadien innerhalb einer Stunde zugetropft. Auf 3 mol der Verbindung der Formel I werden 21 mol Permanganat-Ionen verwendet. Die Temperatur des Reaktionsgemisches wird durch Kühlung mit Eiswasser auf 20 °C gehalten, anschließend wird noch eine Stunde bei Raumtemperatur gerührt. Nun wird auf 50 °C erwärmt und das gebildete Mangan-IV-oxid unter Verwendung von Unterdruck abfiltriert. Der Filterkuchen wird mit 100 $cm^3$ Wasser von 80 °C gerührt und nochmals mittels Unterdruck abfiltriert. Die vereinigten Filtrate werden durch vorsichtige Zugabe von Schwefelsäure bis zum Erreichen eines pH von 1 angesäuert, auf 20 °C gekühlt und 6mal mit je 100 ml Diethyhlether ausgeschüttelt. Die vereinigten Etherextrakte werden sofort am Rotationsverdampfer möglichst vollständig von Ether befreit. Hierbei soll möglichst schnell gearbeitet werden, da Perfluordicarbonsäuren beim längeren Stehen in Etherlösung unter Etherspaltung in ihre Ethylester übergehen. Der Rückstand wird nach Zusatz von 50 $cm^3$ konzentrierter Schwefelsäure bei einem Druck von 2 Pa einer Kurzwegdestillation unterworfen. Das so erhaltene Produkt wird nach Zugabe von 10 $cm^3$ wasserfreier Schwefelsäure bei einem Druck von 0,1 Pa unter völligem Ausschluß von Feuchtigkeit sublimiert. Es werden 30,5 g Dodecafluorhexan-1,6-dicarbonsäure als weißer Feststoff, der einen Schmelzpunkt von 142 bis 144 °C aufweist und äußerst hygroskopisch ist, erhalten. Die Ausbeute beträgt 78,2 %.

Vergleichsversuch A

Analog der aus JP-AS 68-29,129 bekannten Methode soll versucht werden, Perfluornonansäure herzustellen. Hierzu wird in einer 500 $cm^3$ fassenden Rührapparatur mit Rückflußkühler unter kräftigem Rühren zu einer 90 ° heißen Mischung aus 55,44 g (0,35 mol) Kaliumpermanganat und 150 $cm^3$ entsalztem Wasser Perfluoroctylethen getropft. Es findet zunächst keine erkennbare Reaktion statt. Nachdem etwa 15 g des Perfluoroctylethens zugegeben sind, springt die Reaktion plötzlich so heftig an, daß der Kolbeninhalt rasch durch den Kühler ins Freie gedrückt wird. Der Versuch wird daraufhin abgebrochen.

Die Differential-Thermoanalyse einer Mischung von Kaliumpermanganat, Wasser und Perfluoroctylethen im Gewichtsverhältnis 1,12 : 3,81 : 1 ergibt erst ab 400 °C eine exotherme Reaktion.

**Patentansprüche**

1. Verfahren zur Herstellung von Perfluorcarbonsäuren durch Oxidation von Perfluoralkylalkenen mit Permanganat in wäßriger Lösung, gegebenenfalls bei erhöhter Temperatur, dadurch gekennzeichnet, daß man 1/p mol von einer Verbindung der Formel

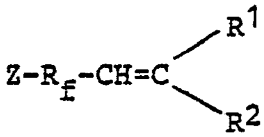 (I),

worin bedeuten
$R_f$ eine geradkettige, verzweigte oder cyclische Perfluoralkylengruppe mit 6 bis 18 C-Atomen;
Z = F; Cl; Br oder

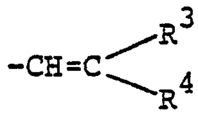

$R^1$, $R^2$, $R^3$ und $R^4$ jedes für sich ein Wasserstoffatom oder eine Alkylgruppe mit 1 bis 5 C-Atomen, wobei ferner
p die Anzahl der Doppelbindungen im Molekül der Formel I ist,
in Gegenwart von 0,001 bis 0,1 mol mindestens eines Alkyl- und/oder Arylalkyl-Gruppen enthaltenden quaternären Ammoniumsalzes bei 10 bis 100°C umsetzt, nach Beendigung der Reaktion die Mischung ansäuert, das entstandene Mangan-IV-oxid abtrennt oder durch Reduktion zum Mangan-II-salz in Lösung bringt, die entstandene Perfluormono- oder -di-carbonsäure abtrennt und, sofern erforderlich, weiter reinigt.

2. Verfahren nach Anspruch 1, dadurch gekennzeichnet, daß Natriumpermanganat und/oder Kaliumpermanganat eingesetzt wird.

3. Verfahren nach Anspruch 1 oder 2, dadurch gekennzeichnet, daß eine Verbindung der Formel I eingesetzt wird, in der bedeuten:
Z = F, Cl oder Br sowie $R^1$ und $R^2$ = H, wobei auf 3 mol dieser Verbindung mindestens 10 mol Permanganat-Ionen verwendet werden.

4. Verfahren nach Anspruch 1 oder 2, dadurch gekennzeichnet, daß eine Verbindung der Formel I eingesetzt wird, in der bedeuten:
Z = $-CH=CH_2$ sowie $R^1$ und $R^2$ = H, wobei auf 3 mol dieser Verbindung mindestens 20 mol Permanganat-Ionen verwendet werden.

5. Verfahren nach Anspruch 1 oder 2, dadurch gekennzeichnet, daß eine Verbindung der Formel I eingesetzt wird, in der bedeuten:
Z = F, Cl oder Br sowie $R^1$ und $R^2$ = Alkyl mit 1 bis 5 C-Atomen, wobei auf 1 mol dieser Verbindung mindestens 2 mol Permanganat-Ionen verwendet werden.

6. Verfahren nach Anspruch 1 oder 2, dadurch gekennzeichnet, daß eine Verbindung der Formel I eingesetzt wird, in der bedeuten:

Z = 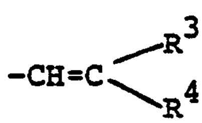

sowie $R^1$,$R^2$, $R^3$ und $R^4$ unter sich gleich oder verschieden = Alkyl mit 1 bis 5 C-Atomen, wobei auf 1 mol dieser Verbindung mindestens 4 mol Permanganat-Ionen verwendet werden.

7. Verfahren nach einem oder mehreren der Ansprüche 1 bis 6, dadurch gekennzeichnet, daß als quaternäres Ammoniumsalz mindestens eine Verbindung der Formel

$$[(R^5)_2(R^6)_2N]^+ X^- \text{ und } [(R^5)_3R^6N]^+ X^-,$$

in denen bedeuten:
$R^5$ einen Alkyl- oder Arylalkylrest mit 8 bis 20 C-Atomen
$R^6$ einen Alkylrest mit 1 bis 3 C-Atomen
$X^-$ den 1/m-ten Teil eines m-wertigen Ions, ausgewählt aus der Gruppe: $Cl^-$; $Br^-$; $R^8SO_4^-$; $SO_4^{--}$; $PO_4^{---}$; $R^8PO_4^{--}$; $(R^8)_2PO_4^-$; $R^7SO_3^-$; $R^7PO_3^{--}$; $(R^7)_2PO_3^-$, . worin bedeuten
$R^7$ = Alkyl- mit 1 bis 4 C-Atomen
$R^8$ = H oder Alkyl- mit 1 bis 4 C-Atomen
eingesetzt wird.

8. Verfahren nach einem oder mehreren der Ansprüche 1 bis 7, dadurch gekennzeichnet, daß nach Beendigung der Reaktion das gebildete Mangan-IV-oxid bei einem pH-Wert der Reaktionsmischung von 0 bis 6,5 zu einer wasserlöslichen Mangan-II-Verbindung reduziert wird.

9. Verfahren nach Anspruch 8, dadurch gekennzeichnet, daß als Reduktionsmittel Schwefeldioxid verwendet wird, das gasförmig dem Reaktionsgemisch zugegeben oder im Reaktionsgemisch gebildet wird.

## Claims

1. A process for the preparation of perfluorocarboxylic acids by oxidising perfluoroalkylethylenes with permanganate in aqueous solution, if appropriate at an elevated temperature, characterised by reacting 1/p mol of a compound of the formula

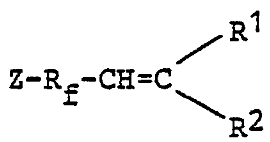

(I)

in which

$R_f$ denotes a linear, branched or cyclic perfluoroalkylene group having 6 to 18 carbon atoms;

Z denotes F, Cl, Br, or

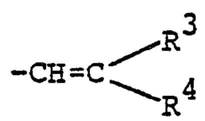

and

$R^1$, $R^2$, $R^3$, and $R^4$ each denote a hydrogen atom or an alkyl group having 1 to 5 carbon atoms, and moreover

p is the number of double bonds in the molecule of the formula I,

in the presence of 0.001 to 0.1 mol of at least one quaternary ammonium salt containing alkyl and/or arylalkyl groups at 10 to 100°C, acidifying the mixture after the completion of the reaction, removing the manganese-IV oxide formed or bringing it into solution by reduction to give the manganese-II salt, and separating off and, if required, purifying further, the resulting perfluoromonocarboxylic or perfluorodicarboxylic acid.

2. The process as claimed in claim 1, characterised in that sodium permanganate and/or potassium permanganate is employed.

3. The process as claimed in claim 1 or 2, characterised in that a compound of the formula I in which Z denotes F, Cl, or Br and $R^1$ and $R^2$ denote H is employed, at least 10 mol of permanganate ions being used for 3 mol of this compound.

4. The process as claimed in claim 1 or 2, characterised in that a compound of the formula I in which Z denotes $-CH=CH_2$ and $R^1$ and $R^2$ denote H is employed, at least 20 mol of permanganate ions being used for 3 mol of this compound.

5. The process as claimed in claim 1 or 2, characterised in that a compound of the formula I in which Z denotes F, Cl, or Br and $R^1$ and $R^2$ denote alkyl having 1 to 5 carbon atoms is employed, at least 2 mol of permanganate ions being used for 1 mol of this compound.

6. The process as claimed in claim 1 or 2, characterised in that a compound of the formula I in which Z denotes

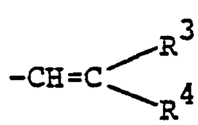

and $R^1$, $R^2$, $R^3$, and $R^4$ are identical with one another or different and denote alkyl having 1 to 5 carbon atoms is employed, at least 4 mol of permanganate ions being used for 1 mol of this compound.

7. The process as claimed in one or more of claims 1 to 6, characterised in that at least one compound of the formula

$$[(R^5)_2(R^6)_2N]^+X^- \quad \text{and} \quad [(R^5)_3R^6N]^+X^-$$

in which

$R^5$ denotes an alkyl or arylalkyl having 8 to 20 carbon atoms,

$R^6$ denotes an alkyl radical having 1 to 6 carbon atoms and

X– denotes the 1/mth part of an m-valent ion selected from the group: $Cl^-$; $Br^-$; $R^8SO_4^-$; $SO_4^-$; $PO_4^{---}$; $(R^8)_2PO_4^{--}$; $R^7SO_3^-$; $R^7PO_3^{--}$ or $(R^7)_2PO_3^-$

in which
$R^7$ denotes alkyl having 1 to 4 carbon atoms and
$R^8$ denotes H or alkyl having 1 to 4 carbon atoms,
is employed as a quaternary ammonium salt.

8. The process as claimed in one or more of claims 1 to 7, characterised in that after the completion of the reaction, the manganese-IV oxide formed is reduced at a pH of 0 to 6.5 in the reaction mixture to give a water-soluble manganese-II compound.

9. The process as claimed in claim 8, characterised in that the reducing agent used is sulfur dioxide, which is added in the form of gas to the reaction mixture or is formed in the reaction mixture.

**Revendications**

1. Procédé pour préparer acides perfluorocarboxyliques, par oxydation de perfluoro-alkyl-alcènes avec du permanganate en solution aqueuse, éventuellement à température élevée, procédé caractérisé en ce qu'on fait réagir 1/p mole d'un composé de formule

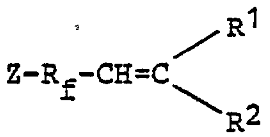

(I),

(dans laquelle
$R_f$ représente un groupe perfluoroalkylène linéaire, ramifié ou cyclique comportant 6 à 18 atomes de carbone;
Z représente F, Cl, Br ou

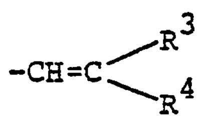

$R^1$, $R^2$, $R^3$ et $R^4$ représentent chacun un atome d'hydrogène ou un groupe alkyle ayant 1 à 5 atomes de carbone,
p représente le nombre de doubles liaisons dans la molécule de formule I),
en présence de 0,001 à 0,1 mole d'un sel d'ammonium quaternaire contenant au moins un groupe alkyle et/ou arylalkyle, à 10 jusqu'à 100°C, après achèvement de la réaction on acidifie le mélange, on sépare l'oxyde de manganèse-IV résultant ou bien on le fait passer en solution par réduction en un sel de manganèse-II, on sépare l'acide perfluoromono- ou di-carboxylique résultant et, si nécessaire, on le soumet à une purification supplémentaire.

2. Procédé selon la revendication 1, caractérisé en ce qu'on utilise du permanganate de sodium et/ou du permanganate de potassium.

3. Procédé selon la revendication 1 ou 2, caractérisé en ce qu'on utilise un composé de formule I, dans lequel Z représente F, Cl ou Br et $R^1$ et $R^2$ représentent chacun H et, pour 3 moles de ce composé, on utilise au moins 10 moles d'ions permanganate.

4. Procédé selon la revendication 1 ou 2, caractérisé en ce qu'on utilise un composé de formule I, dans lequel Z représente $-CH=CH_2$, et $R^1$ et $R^2$ représentent chacun H et, pour 3 moles de ce composé, on utilise au moins 20 moles d'ions permanganate.

5. Procédé selon la revendication 1 ou 2, caractérisé en ce qu'on utilise un composé de formule I, dans lequel Z représente F, Cl ou Br et $R^1$ et $R^2$ représentent chacun un groupe alkyle ayant 1 à 5 atomes de carbone et, pour 1 mole de ce composé, on utilise au moins 2 moles d'ions permanganate.

6. Procédé selon la revendication 1 ou 2, caractérisé en ce qu'on utilise un composé de formule I, dans lequel Z représente

Z = 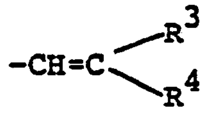

et $R^1$, $R^2$, $R^3$ et $R^4$ sont identiques ou différentes et représentent chacun un groupe alkyle ayant 1 à 5 atomes de carbone et, pour 1 mole de ce composé, on utilise au moins 4 moles d'ions permanganate.

7. Procédé selon une ou plusieurs des revendications 1 à 6, caractérisé en ce qu'on utilise comme sels d'ammonium quaternaire au moins un composé de formules

$$[(R^5)_2(R^6)_2N]^+X^- \quad \text{et} \quad [(R^5)_3R^6N]^+X^-,$$

dans lesquelles
$R^5$ représente un reste alkyle ou arylalkyle ayant 8 à 20 atomes de carbone,
$R^6$ représente un reste alkyle ayant 1 à 3 atomes de carbone,
$X^-$ représente la 1/m partie d'un ion de valence m, choisi parmi $Cl^-$, $Br^-$, $R^8SO_4^-$, $SO_4^{--}$, $PO_4^{---}$, $R^8PO_4^{--}$, $(R^8)_2PO_4^-$, $R^7SO_3^-$, $R^7PO_3^{--}$, $(R^7)_2PO_3^{--}$, où
$R^7$ représente un groupe alkyle ayant 1 à 4 atomes de carbone et
$R^8$ représente H ou un groupe alkyle ayant 1 à 4 atomes de carbone.

8. Procédé selon une ou plusieurs des revendications 1 à 7, caractérisé en ce que, après achèvement de la réaction, on réduit à un pH du mélange réactionnel de 0 à 6,5, l'oxyde de manganèse-IV formé en un composé de manganèse-II hydrosoluble.

9. Procédé selon la revendication 8, caractérisé en ce qu'on utilise comme réducteur du bioxyde de soufre que l'on ajoute sous forme de gaz au mélange réactionnel ou que l'on forme dans le mélange réactionnel.